# EUROPEAN PATENT SPECIFICATION

(11) **EP 4 143 834 B1**
(45) Date of publication and mention of the grant of the patent: **18.03.2026**
(21) Application number: 21722872.5
(22) Date of filing: 30.04.2021
(51) Int. Cl.: G16H 10/60, G16H 40/20, G16H 10/20, G16H 20/10

(54) **A MEDICAL MANAGEMENT SYSTEM AND A METHOD THEREOF**
MEDIZINISCHES VERWALTUNGSSYSTEM UND VERFAHREN DAFÜR
SYSTÈME DE GESTION MÉDICALE ET PROCÉDÉ ASSOCIÉ

(30) Priority: 01.05.2020 GB 202006456; 19.06.2020 IE S20200136
(43) Date of publication of application: 08.03.2023
(73) Proprietor: HealthBeacon PLC, D12WD85 Dublin 12 (IE)
(72) Inventor: JOYCE, Jim, Dublin, WD85 (IE); DALY, Kieran, Dublin, WD85 (IE); SHATTOCK, Richard, Dublin, WD85 (IE)
(74) Representative: Murgitroyd & Company
(86) International application number: PCT/EP2021/061472
(87) International publication number: WO 2021/219885

(56) References cited:
- WO-A1-2017/035474
- WO-A1-2020/049325
- US-A1- 2014 374 294
- US-A1- 2017 300 659

## Description

### Field

The present application relates to a system and method for managing and tracking medical and/or clinical events associated with a patient. More specifically, the present invention relates to a system and method for verifying and validating medical and/or clinical events such as a therapeutic drug administration, a vaccine administration against a pathogen, or medical testing of the patient.

### Background

Vaccination is one of the most effective ways to prevent diseases. A vaccine helps the body's immune system recognise and fight pathogens like viruses or bacteria, which keeps us safe from the diseases they cause. Vaccines protect against a range of debilitating and/or life-threatening diseases, including measles, polio, tetanus, diphtheria, meningitis, influenza, tetanus, typhoid and cervical cancer.

In addition to routine vaccination such as seasonal flu vaccinations, organisations need to have an up-to-date record of vaccination statistics to control the risk of a specific disease effectively. For example, when an effective vaccine has been developed to battle the current global Covid-19 pandemic, the general public may be encouraged or required to receive vaccinations to control the spread and lower the risk of the disease. Given the current Covid-19 pandemic and future such pandemics, it will become a requirement to demonstrate that individuals have been vaccinated so as to have achieved immunity. From the perspective of individuals, patients are generally underinformed as to what immunisations are recommended or on what schedule they should be received to achieve immunisation. Few patients are aware of or understand the spectrum of recommended immunisations. Dosing schedules are outlined in the Summary of Product Characteristics (SPC) for each vaccine, which provide details on how these should be spaced depending on the clinical trial data. It has also become apparent that during times of mass vaccination and reducing community-based transmission that the dosing schedule may also be altered based on advice from Government and Medical experts. Instead, patients have to rely on medical providers to ensure adherence to recommended immunisation schedules. However, it may become challenging for patients to adhere to a recommended vaccine administration course or for administrators to appropriately manage patient health record by updating their immunisation details on a local immunisation register. As a result, it is difficult with existing solutions to ensure that patients adhere to recommended vaccination course and further manage the individual health records.

Similarly, in the case of other medical and/or clinical events such as drug trial, test sample collection, administration of injectable medicines, it is critical that the patients adhere to their prescribed schedule. As such, it is essential to monitor and manage medical and/or clinical events associated with the patient to ensure that they adhere to their prescribed schedule.

Therefore, there is a need for providing a medical care management system for managing, tracking, and validating medical events such as vaccine administration, drug trials, test sample collection, the administration of a medicament, and the like.
Reference is made to the documents US 2014/374294 A1, WO 2017/035474 A1, WO 2020/049325 A1 and US 2017/300659 A1 cited as relating to the state of the art.

### Summary of the invention

The scope is in accordance with the appended claims.

### Summary of related disclosure

An aim of the present disclosure is to provide a medical management system and a method to manage, monitor, and validate, medical events associated with patients. For example, the present disclosure may be used to manage, monitor, and validate vaccine administration events, collection and tracking of medical test samples and results reporting, administration of a medicament e.g. therapeutic drug, contact tracing of individuals to a particular pathogen, verification of a medical event, and the like. In general, the present disclosure provides a centralised system and method for monitoring, tracking, and validating medical and/or clinical events associated with individual patents such as pathogen care events, and the like.

In accordance with the present disclosure, the term pathogen care management system encompasses any system that is capable of monitoring, managing, and verifying a medical and/or clinical event e.g. such as the administration of injectable drugs for a particular disease associated with the patient such as crohn's disease, diabetes, a drug trial, and the like. Furthermore, the pathogen care management system may be used for the collection of test samples e.g. blood samples, reporting of results to patients, and the like. As such the pathogen care management system of the present invention may also be referred to as a medical or clinical management system.

In accordance with the present disclosure, the term pathogen care item may be any medical and/or clinical item associated with a medical and/or clinical event such as a vaccine sharp and/or via, an injectable medication or a particular disease, a test sample, and the like.

In accordance with the present disclosure, the pathogen care software application may be any type of software application that can be used to monitor and manage medical and/or clinical events such as vaccine administration, test sample collection, drug trial, administration of an injectable medication to a patient for a particular disease, and the like. Different versions of the software may be provided to patients and the healthcare professionals associated with the medical and/or clinical event. In other embodiments, the software application may be the same but with different access levels. In any case, the software application runs on corresponding electronic devices associated with the patients and the healthcare professionals. Depending on the setting, the patient may operate the software application as an administrator e.g. the medical event takes places at the home of the patient. In other cases, the healthcare professional may be provided with administrator rights and the patient may be provided with a different version of the software application.

Accordingly, the present invention provides a system and a method as detailed in the independent claims. Advantageous embodiments are provided in the dependent claims.

According to a first aspect of the present invention, a pathogen care management system is provided according to claim 1.

It has been found that the use of a centralised PCM platform for managing and monitoring pathogen care events such as vaccine administration events and the like offers the possibility of accurately track and monitor parameters associated with the pathogen care event and the user participating in the pathogen care event. For example, with the use of the PCM platform, the users are provided with a digital health record that comprises information about the health history of the user, including information on the pathogen care event, e.g. vaccine administration, tests performed, and the like. The PCM platform is configured to receive information from a plurality of devices associated with the pathogen care management system. For example, the PCM platform may receive information from an electronic device associated with an administrator of the pathogen care event, a pathogen care device configured to detect the deposit of a pathogen care item, an electronic device of a user, and the like. The PCM platform may receive information from different devices and, accordingly, based on information transmitted, update the digital health record of the individual patients participating in the pathogen care event. For example, in a vaccination event deploying multiple pathogen care devices over multiple sites, the PCM platform may collect information associated with each user participating in the vaccination from electronic devices associated with the administrator and corresponding pathogen care devices. The digital health record of each user is updated based on the information transmitted, and the user is able to access the relevant information.

According to embodiments of the present disclosure, the processing unit of the PCM platform comprises a validation module configured, before updating a digital health record, to validate the corresponding pathogen care event by comparing information contained in the pathogen care data record data and/or user identification data record with reference information retrieved from a database of the data storage module and/or stored in the digital health record of the corresponding user.

According to embodiments of the present disclosure, in response to a valid pathogen care event, the processing module is configured to update the health status of the user digital health record and issue a corresponding certificate, which is stored in the digital health data record of the user and further transmitted to a user software application running on an electronic device associated with the user.

According to embodiments of the present disclosure, in response to an invalid pathogen care event, the processing module is configured to update the health status of the corresponding digital health record with the rejection notification.

According to embodiments of the present disclosure, in response to a rejection notification, the processing unit is configured to select from a database of the data storage module one or more procedures to be followed, each at least comprising the issuance of a set of notifications to one or more register users and/or external systems.

The processing unit of the PCM platform is configured to validate the information associated with a pathogen care event. For example, the processing unit may be provided with a validation module configured to compare the information received from at least one electronic device and a pathogen care device with reference information. For example, the validation processing unit may compare information associated with the pathogen care item given to the user with reference information, e.g. stored in the digital health record of the user, to determine whether the correct pathogen care item was administered during the pathogen care event. Other information contained in the user identification and/or pathogen care data records may be used to validate the pathogen care event, such as proximity of an electronic device associated with the user device to the pathogen care device and/or electronic device of the administrator, confirmation received from an administrator, user identification information, and the like. The proximity of the user's electronic device may be assessed in various ways, such as the scanning of an identification code presented at the screen of the electronic device associate with a user, pairing through a short-range wireless connection, and the like. The user identification information may comprise biometric features of the user, which are compared with stored biometric features, e.g. fingerprint, facial features, and the like. The validation module is configured to issue a certificate associated with the pathogen care event, e.g. a vaccination certificate if the transmitted information is valid. Otherwise, the validation module issues a rejection notification and updates the status of the digital health record. In response to issuing a rejection notification, the validation module may select a follow-up procedure identifying the next steps for correcting the invalid pathogen care event. For example, if the wrong pathogen care item was given to the patient, the validation module may issue a notification to the user with a recommended set of actions which are defined based on the severity of the situation, e.g. go to the hospital, repeat the pathogen care event, and the like.

The processor unit may be configured to assign a confidence score to the health status of the digital health record based on a set of criteria comprising any one of the period between pathogen care events, a physical location of the user, or adherence of the patient to a pathogen care medical course. For example, the pathogen status of the patient may be assigned a confidence score, which may indicate the risk of the individual becoming susceptible to a particular pathogen, e.g. particular disease. As such, the risk associated with a patient being sick with a particular pathogen may be assessed. For example, a patient that has not received a vaccine for a virus has been present at a virus affected location or in contact with a virus carrying person would be more likely to be carrying the virus, even if the pathogen status indicates that the patient is healthy. Based on the confidence score, specific actions may be initiated for the individual patient, e.g. take a medical test, vaccine administration, quarantine, and the like. The confidence score may be used to manage and prevent the spreading of a pathogen to the community.

According to embodiments of the present disclosure, the processing unit comprises a reminder module configured to issue one or more reminders associated with a pathogen care event, based on information contained in the corresponding user digital health records and/or information received from an external system, the reminders being transmitted to one or more electronic devices associated with a user at a selected frequency.

According to embodiments of the present disclosure, the reminder module is configured to adjust the reminder transmission frequency based on a classification of the reminders into normal, urgent, and emergency types. For example, depending on the type of reminder, the user may receive one or more reminders. The classification of the reminders may be performed based on a set of predetermined criteria, such as criticality of the event, a trigger event source of information. The critically is directly associated with the information received from the electronic device, the pathogen care device, and/or another source, e.g. health authority. For example, if the patient was given the wrong medication or health authority has identified a risk with a specific medicament, this event may be considered an emergency, and the reminders may be set as such, e.g. one every hour. On the other hand, an appointment for vaccination may be considered a normal reminder, and the frequency may be adjusted accordingly, e.g. once a day. In addition, the frequency and number of the notification may increase as the date of an event comes closer. The reminder module may be configured to send reminders to patients and/or administrators, based on an adherence schedule associated with a specific medical and/or clinical event. For example, the reminder module may be configured to send reminders to a patient associated with a scheduled appointment for a vaccination. The reminders may become more frequent as the date of the appointment is approaching.

According to embodiments of the present disclosure, the pathogen care data record comprises information obtained from an image of the pathogen care item and/or scanning of an identification code associated with the pathogen care item. For example, the pathogen care device may be configured to obtain information from an image of the pathogen care item and scan an ID code such as bar code, QR code, and the like. The information extracted is added to the pathogen care data record, e.g. batch number of a vaccine, type of vaccine, and the like. An image of the pathogen care item may also be added to eh pathogen care data record, which is processed by the processing unit of the PCM platform.

According to embodiments of the present disclosure, the pathogen care item is a vaccine sharp and/or a vaccine vial. The pathogen care item may, in general, be any one of a vaccine sharp, a medication sharp, a vial containing the medicament administered during the pathogen care event, a test sample obtained from the user deposited in the pathogen care device to be transferred to an analysis lab, and the like.

According to embodiments of the present disclosure, the pathogen care software application is configured to identify a user based on a biometric ID of the user comprising at least one biometric feature associated with the user. In general, the user may be identified based on biometric features extracted from a scanned image, such as facial features, fingerprints, and the like. Other ways may also be used to identify the user, such as processing information on an identification document such as a passport, ID cards, and the like. For example, at least one first electronic device is configured to identify a patient based on secure registration, which could include a biometric ID of the patient comprising at least one biometric feature associated with the patient. The biometric features may include but not limited to fingerprints, iris, face or security code delivered via SMS or email, and the like. For example, the electronic device may be a mobile phone comprising a facial recognition or fingerprint recognition software capable of obtaining and optionally validating a user ID.

According to embodiments of the present disclosure, the pathogen care software application is configured to identify a user based on scanning an identification code presented on a screen of an electronic device associated with the user. Furthermore, the user may be identified based on scanning an identification code displayed on the electronic user device, such as a QR code sent to the user before the pathogen care event and containing information on their registration.

According to embodiments of the present disclosure, the processing unit comprises a dispensation management module configured to activate dispensation of a pathogen care item from a storage receptacle, based on information contained in at least the user identification data record. For example, the processing unit may be communicatively coupled to a storage receptacle that stores the pathogen care items, e.g. a fridge. The storage receptacle may be part of the pathogen care device or maybe a separately connected unit. Monitoring dispensation ensures that pathogen care items are dispensed only for authorised events and further monitor the inventory of the pathogen care items at the different locations.

According to embodiments of the present disclosure, the dispensation management module is configured to monitor a set of parameters of the storage receptacle.

According to embodiments of the present disclosure, the dispensation management module is configured, in response to at least one parameter value being within a threshold value, to register the event in the data storage module and issue one or more notifications.

According to embodiments of the present disclosure, the set of parameters comprises any one of or a combination of a storage temperature, pathogen care items stock level, the time elapsed between the dispensation of pathogen care item and detection of one or more corresponding pathogen care items by the pathogen care device.

According to embodiments of the present disclosure, in response to the elapsed time between dispensation and detection of a pathogen care item being within a threshold value range, the dispensation management module is configured to issue one or more reminders to the electronic device associated with the administrator.

According to embodiments of the present disclosure, the dispensation management module is configured to increase the frequency and number of reminders issued in proportion to the length of time elapsed between the dispensation of a pathogen care item and detection of one or more corresponding pathogen care items by the pathogen care device.

According to embodiments of the present disclosure, in response to the stock level of the pathogen care items being within a first threshold value, the dispensation management module is configured to issue one or more notifications to an order management system for the delivery of new pathogen care items to the storage receptacle.

According to embodiments of the present disclosure, the processing unit comprises an order monitoring module configured to monitor the delivery of pathogen care items to the storage receptacle, based on information received from one or more touchpoints associated with the order management system.

For example, the dispensation management module may monitor a range of parameters and accordingly take necessary actions such as issuing a notification when the storage temperature drops below a threshold value, ordering new pathogen care items when the inventory is low, or monitoring the amount of time elapsed between a pathogen care item being dispensed and a corresponding pathogen care item being detected by the pathogen device. For example, in the case of a vaccine or another medicament, the medicament may need to be administered to the patient within a certain amount of time before it becomes unusable or less effective, e.g. self-life deteriorates the longer it is maintained at room temperature. As such, the dispensation module may be configured to increase the frequency and number of notifications issued as the elapsed time between dispensation and use of the pathogen care item becomes longer. Therefore, it is crucial to be able to monitor such events and accordingly issue a notification to inform a registered user, e.g. administrator. Furthermore, the processing unit may be configured to monitor whether the pathogen care item dispensed was used in accordance with a predetermined protocol. For example, a vaccine or medicament vial may be used to extract a plurality of doses, and each dose being administered using corresponding pathogen care items, e.g. a single vial may be used to administer a number of vaccines to corresponding users. As such, the processing unit may monitor the number of dosages administered before dispenses another pathogen care vial. Furthermore, the dispensation management module is configured to manage the inventory of the pathogen care items and, based on the stock levels, issue notification to an order management system for ordering new pathogen care items. The dispensation module monitors the delivery of the pathogen care items based on information received from different touchpoints of the order management system. In this way, the present invention manages the inventory, prevents authorised use of the pathogen care items, and prevents improper use of the pathogen care items.

According to embodiments of the present disclosure, the processing unit comprises a waste management module configured to monitor a capacity parameter of the storage compartment of at least one pathogen care device indicating an available space for receiving pathogen care items, which volume parameter is calculated based on information associated with the detected pathogen care item placed in the storage compartment.

According to embodiments of the present disclosure, in response to the capacity parameter being within a threshold value range, the waste management module is configured to issue a waste collection notification to a waste collection system communicatively coupled to the PCM platform.

According to embodiments of the present disclosure, the waste notification comprises information associated with the geographic location of at least one pathogen care device and information associated with the type of pathogen care items contained in the storage compartment.

According to embodiments of the present disclosure, the waste collection system is configured to activate a waste collection service and further select, based on the identified pathogen care item types and/or type of pathogen care event, one or more a waste disposal procedures for processing the pathogen care items in the storage compartment.

The present disclosure further enables the automatic collection of the deposited pathogen care items. For example, the pathogen care management system may comprise a waste management collection system, which is configured to process the pathogen care items placed in the storage compartment of each pathogen care device. A waste management module of the PCM platform may monitor the available space of each connected pathogen care device, e.g. based on signal issued by the pathogen care devices, and accordingly, issue a collection notification to the waste collection management system. Furthermore, the waste collection system may select, based on the type of pathogen care items in the storage compartment of each pathogen care device, a specific waste processing procedure, e.g. recycling, incineration, and the like.

According to embodiments of the present disclosure, at least one pathogen care device is a sharps bin comprising:
a removable storage compartment configured to receive pathogen care item deposits;
at least one sensor module configured to detect the pathogen care item deposit;
a timestamping module configured to timestamp the pathogen care item deposit;
a processing module for generating the pathogen care data record; and
a communication module configured for transmitting the pathogen care data record to the PCM platform.

According to embodiments of the present disclosure, the pathogen care event comprises any one of a therapeutic drug administration, a vaccine administration against a pathogen, or an extraction of a medical test sample from the user to be analysed for the detection of at least one pathogen.

According to a second aspect of the present invention, a method for recording and validating pathogen care events associated with users according to claim 13 is provided.

In general, the present disclosure enables the management and monitoring/tracking of pathogen care events associated with a patient. Furthermore, the present disclosure enables patients to create a centralised digital health record, which may store a variety of medical-related information, e.g. medical history, vaccination log keeping track of the vaccine administration events, upcoming pathogen care events, and the like. Furthermore, the present disclosure may enable consent and identification of each individual patient, verification and identification of the pathogen care event and corresponding pathogen care item, e.g. vaccine (make/batch). For example, the present disclosure may detect and digitally verify disposal of a vaccine vial/needle, the placement of a test sample vial in the pathogen care device, and the like. The present disclosure provides a robust means of patient identification and consent associated with the corresponding pathogen care event through biometric information such as fingerprint scanning obtained through the pathogen care software application running on an electronic device, e.g. the mobile phone of the patient. The detection of the pathogen care item provides a pathogen care digital record containing a variety of information. For example, the pathogen care digital record may contain information on the drug administered, time of administration, a verification by healthcare professionals that a drug was administered, information on a test sample via such as type of analysis to be performed, and the like. Furthermore, the present disclosure enables efficient and accurate confirmation of the occurrence of pathogen care events. For example, the present invention enables effective validation of pathogen care events by establishing the physical proximity of the patient or a device associated with the patient to the pathogen care device during the pathogen care event to enhance the accuracy of confirmation and validating of vaccinations. The physical proximity information may be performed by, but not limited to: physical location of the patient established through the geolocation of the electronic device associated with the patient, a connection established between the electronic device associated with the patient with the pathogen care device, the verification by a healthcare professional and the like. The proximity information is included in the pathogen care digital record transmitted to the pathogen care management platform.

Further technical advantages of the system and method include but not limited to:
- providing real-time data for the uptake and effectiveness of immunisation programmes. For example, the PCM platform may provide information on the uptake of immunisation programmes launched by a governmental health authority, based on data contained in the digital health records associated with a patient,
- reminding patients when a pathogen care event is due to take place and monitor adherence of the patient to the pathogen care event. For example, the present invention may issue reminders when a vaccine administration is due or when new vaccines are available, which provides further protection against new strains of the pathogen. The notifications may be extended to include other types of reminders, e.g. conducting a medical test, health authority notifications, and the like.
- Managing the availability and stock of the pathogen care items associated with the pathogen care events. Furthermore, the present disclosure may be configured to manage dispensation of the request pathogen care item based on the credentials of an authorised user. For example, the present disclosure may manage the stock and dispensation of vaccines or other therapeutic drugs. As a result, the present disclosure may prevent counterfeiting or incorrect pathogen care item from being used in pathogen care events.
- Enable the stock management and supply chain monitoring. For example, the present disclosure may place orders for pathogen care items in an order system when the stock of the pathogen care item reaches a threshold.

Furthermore, the present disclosure may enable tracking the pathogen care
item along the supply chain channels, from ordering to delivery, based on information received from electronic checkpoints along the supply chain. For example, the PCM platform may be connected to electronic checkpoints associated with different stages along the supply chain, e.g. manufacturer, supplier, delivery, and the like. Therefore, it becomes possible to monitor and track the journey of pathogen care items, which may prevent counterfeiting and further enable easy management of mass population immunisation. Likewise, with a diagnostic pathogen event, the system may track various stages of the process from scheduling the test procedure, sample extraction, purification, analysis and reporting.
- Improving community-based pharmacy administration and reduce record and paperwork keeping. The PCM system may be a portable system that may be used transported and used in a diverse range of locations, e.g. a patient's home, a pharmacy, a clinic and the like. Furthermore, the patient may use the pathogen care software application to fill in all the necessary information and provide consent prior to the pathogen care vaccination event.
- Facilitating reporting to users of adverse events associated with the pathogen care event, e.g. wrong vaccine administered, possible contact with an individual that tested positive to a particular pathogen, vaccine side effects, and the like;
- Facilitating management of large-scale population immunisation vaccinations and reporting the results for analysis.
- Furthermore, the system may also be used during clinical trials to assist with data collection.
- Furthermore, the present disclosure could allow tracking of cold chain stock requiring careful preparation prior to administration, and vaccine vials may need to be adequately stored as well as sufficiently thawed prior to administration. The present disclosure could track and verify that vials were thawed correctly by measuring the time lapse between vaccine batch barcode scanning and the administration event. Furthermore, the present disclosure could allow tracking of vaccine shelf-life once it has been thawed.

Depending on the vaccine type, a vial that has been reconstituted must be used within a short timeframe, perhaps 6 hours. mRNA vaccines may only have a shelf life of 24 hours at -30C to 200C, with higher temperatures reducing the shelf life.

### Brief Description of the Drawings

Figure 1A shows an exemplified implementation of a pathogen care management system in accordance with embodiments of the present invention.
Figure 1B shows a schematic of an exemplified pathogen care device according to embodiments of the present invention.
Figure 2 shows a process flow of an exemplary pathogen care management method for reporting and verifying vaccine administration in accordance with embodiments of the present invention.
Figure 3 shows a process flow of an exemplary pathogen care method for verifying vaccine administrations in accordance with embodiments of the present invention.
Figure 4 shows a process flow of an exemplary pathogen care method for managing a pathogen care item supply chain in accordance with embodiments of the present invention.
Figure 5 shows an example of the system illustrated in figure 1 for monitoring the occurrence of a vaccine administration event at least involving administering a vaccine to a user according to a prescribed vaccination course.
Figure 6 shows an exemplified pathogen care method for a vaccine administration event according to embodiments of the present invention.
Figure 7 shows an exemplified method for verifying the validity of the vaccine administration event according to embodiments of the present invention.
Figure 8 shows an exemplified architecture of the processing unit of the pathogen care management platform according to embodiments of the present invention.

### Detailed Description

The present invention will now be exemplified with reference to the accompanying drawings. While this invention has been shown and described with reference to certain illustrated embodiments thereof, it will be understood by those skilled in the art that various changes in form and details may be made therein without departing from the scope of the invention encompassed by the appended claims. Furthermore, while the invention has been described with references to a particular system and/or a method for pathogen care management, it should be understood by those skilled in the art that changes in form and details may be made to facilitate other types of method and/or systems used for verifying administration of medicaments, such as injectable medicaments used in chronic conditions.

Throughout the following discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer-readable medium. For example, a server can include one or more computers operating as a web server, database server, or other types of computer server in a manner to fulfil described roles, responsibilities, or functions. One should further appreciate the disclosed computer-based algorithms, processes, methods, or other types of instruction sets can be embodied as a computer program product comprising computer-readable media storing instructions that cause a processor to execute the disclosed steps. One should appreciate that the systems and methods described herein involve interconnected networks of hardware devices configured to receive data using receivers, transmit data using transmitters, and transform electronic data signals.

For simplicity and clarity of illustration, reference numerals may be repeated among the figures to indicate corresponding or analogous elements. Numerous details are set forth to provide an understanding of the examples described herein. The examples may be practised without these details. In other instances, well-known methods, procedures, and components are not described in detail to avoid obscuring the examples described. The description is not to be considered as limited to the scope of the examples described herein.

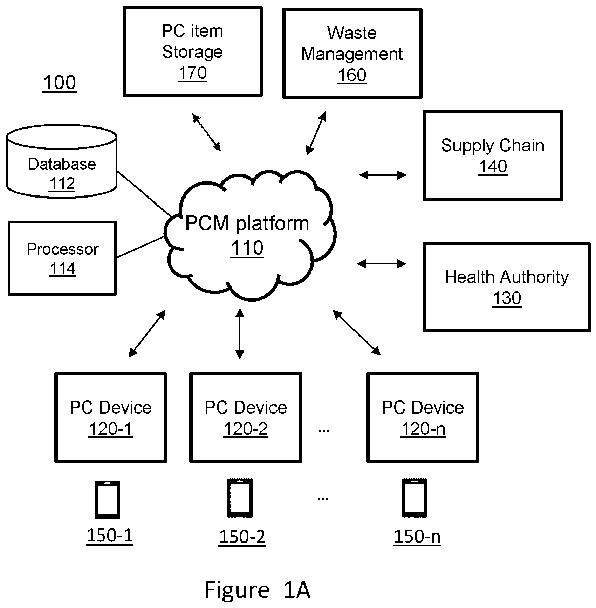

Figure 1A shows an exemplified implementation of a pathogen care management system according to embodiments of the present disclosure The PCM system may be configured for managing and tracking pathogen care events associated with a user. For example, the pathogen care system may be used to track and manage a vaccine administration event, the administration of a therapeutic drug, or a medical test sample. The system comprises at least one pathogen care (PC) devices 120 1-n configured for detecting the use of pathogen care item associated with the pathogen care event. For example, the pathogen care device may be configured for recording deposits of vaccine sharps, i.e. spent vaccine vials after administration of the vaccine, sharps/vials associated with a therapeutic drug, or a medical test sample. Each device may be associated with an authorised user and/or an authorised organisation. For example, each PC device 120 1-n may be associated with a hospital, a clinic, a vaccine centre, a health authority official, a doctor, nurse, or any other user with the credentials for operating the device. Each PC device 120 1-n may be associated with a pathogen care software application that may authenticate the credentials of the user before operating the PC device. The pathogen care software application may authenticate the user's credentials based on a password, a biometric feature of the user, e.g. fingerprint, an electronic signature, or any other known means that can be used for user authentication. For example, the pathogen care software application may be configured to identify a user based on a biometric ID. The software application may be running on the PC devices 120 1-n and/or running on an electronic device 150 1-n, communicatively coupled to the VAV device 120 1-n, e.g. a computer, mobile phone, and the like.

The pathogen care software application may be configured to identify and/or register a user, e.g. a patient, prior to a pathogen care event based on the user's biometric information. For example, the biometric information may be stored as part of the user data record stored either in the electronic device 150 1-n and/or the pathogen care management (PCM) platform. For example, the biometric ID of a user may be stored and/or associated with the digital health record of the user. A user may further use the pathogen care software application to consent to the pathogen care event. For example, the user may consent to the vaccination by, for example, completing a digital consent form via the pathogen care software application. The electronic device 150 1-n may be configured to connect to the PC device 120 1-n to enable the exchange of data. For example, the electronic device 150 1-n may be connected to the PC device 120 via a wireless connection, a Bluetooth, Near Field Communication (NFC), one or more wireless personal area network (WPAN), Wireless local area network (WLAN) or another suitable device-to-device network. The pathogen care device 120 may comprise a proximity module configured to detect when an electronic device 150 is connected/paired with the PC device 120. For example, a user and/or a healthcare professional may be asked when opening the pathogen care software application to allow a connection to a PC device 120, or it may automatically pair when the registered individual comes into close proximity to the PC device 120. For example, the geolocation information of the user may be collected via geolocation API. The proximity information collected from the proximity module may be added to the PC data record.

The pathogen care (PC) device 120 may be configured to detect a pathogen care item associated with the pathogen care event. For example, the pathogen care (PC) device 120 may be configured to detect the deposit of pathogen care items to a storage/collection bin of the pathogen care (PC) device. The pathogen care items may include, but not limited to, vaccine sharps, medical/therapeutic drug vials, test samples and the like. Furthermore, the PC device 120 may be configured to detect the pathogen care items by reading or scanning a label or another device on the pathogen care item. For example, the PC device 120 may comprise an identification device such as a Bluetooth device, GPS device, near field communications module (NFC), bar code scanner, QR code scanner, RFID tag reader and the like. The identification device may be capable of reading and/or obtaining information from a corresponding identification element attached to the pathogen care item. The identification element may be in the form of communication labels: radio/electronic inlays - single or dual frequency NFC/UHF, barcodes, QR codes, digital watermarks, and RFID. The communication labels may provide short- and long-range tracking. For example, short-range (e.g., 1- 4 cm) may be used to track consumer engagement, while long-range (≥ 1 meter) may be used for prescription management/stock control.

For example, the pathogen care (PC) device 120 may be a smart medical storage bin configured to receive pathogen care item deposits. Furthermore, device 120 may be configured to store and/or dispense pathogen care items, e.g. vaccines or associated items (e.g. vaccines, syringes, and the like) for administration to the user as part of a pathogen care event. The PC device 120 may be configured to generate pathogen care (PC) data record, which may be transmitted to the Pathogen Care Management (PCM) platform for storage and further processing. The PC data record may contain a range of information associated with the pathogen care event. For example, the PC data record may contain information associated with the pathogen care item detected by the identification device, such as the type of the pathogen care item, timestamping of the deposit, user details, healthcare professional details, and the like. Furthermore, the PC data record may contain information associated with a dispensation of a pathogen care item. For example, it may contain details of the user requesting the dispensation, a timestamping of the dispensation event, the type of pathogen care item, and the like. As such, data obtained during the dispensation of a requested pathogen care item may be compared to the data retrieved from the deposited pathogen care item.

Furthermore, the PC device 120 and/or pathogen Care Management (PCM) platform may be configured to identify and classify the pathogen care items received. For example, the PC device may be configured to collect the used pathogen care item, e.g. vaccine sharps, and accordingly identify the content of the storage container for downstream recycling processes to aid in the recovery of material from this waste product. For example, in the case of similar type deposited pathogen care items, the PC device 120 may label the storage containers, e.g. using electronic tags, when replaced, so that the content of the storage container may be stored and processed together with other containers containing similar type pathogen care items thus improving downstream recycling process. For example, the PC device 120 may be associated with a specific pathogen care item type, and as such configured to store a predetermined type of pathogen care item, e.g. used vaccine sharps, or therapeutic drug sharps, and the like. Furthermore, the PC device 120 may be provided with a plurality of containers, each configured to store a predetermined type of pathogen care items. The PC device 120 may be provided with a classification device for directing the deposited products to the corresponding storage container. The PC device 120 of the present invention may be used in environmental waste management applications, improving circular economy, product stewardship and Extended Producer responsibility. The PC device 120 may further provide real-time data on waste disposal, identifying, quantifying and measuring consumption during vaccine administration or therapeutic consumption, and providing the data figures for recycling, re-using during waste recovery/processing activities. For example, information associated with identifying the pathogen care items placed in the pathogen care device 120. For example, the information identifying the content of the pathogen care device 120 may include any one of or a combination thereof: component materials plastic, glass, metal, type, grade, weight and the like. The information identifying the content of the storage containers/bin of the PC device 120 may be transmitted to the PCM platform 110 or another suitable system for processing. For example, the identification content information may be part of the pathogen care data record or may be transmitted as a separate data record. For example, based on the detection of the pathogen care items in at least one storage bin of the pathogen care device 120, it may be possible to reconcile the pathogen care items when the storage bin is emptied. Furthermore, the PC device 120 may indicate whether the storage container, also referred to as sharps bin, has reached capacity or is nearing capacity and requires replacement. This can streamline collection and replacement of the PC device (or its internal storage compartment/bin) to facilitate safe sharps management practices as well as further downstream processing of the waste in line with more sustainable waste management initiatives and align with the Government's Climate action plans. The PCM platform 110 may be communicatively coupled to a waste management system 170 configured to collect the storage containers associated with each PC device 120 and accordingly process their respective content. For example, the PCM platform 110 may monitor, based on information received from each PC device 120, the remaining available space in each storage container, and issue a collection notification to the waste management system 170. The waste management system 170 is configured for activating a collection service for the storage container identified in the notification and further select, based on the type of pathogen care item in the container, a disposal procedure, e.g. recycling, incineration, hazardous material processing, and the like. The waste management system 170, which may be part of the pathogen care management system 100, may be configured to assign a carbon figure to each component and output on a dashboard/reporting system to indicate to manufacturers the volume of material recycled from their devices and the carbon savings associated with the recycling activity. The pathogen care items that may be disposed of in the storage container/bin may include but not limited to any one of or combination thereof of the following items:
- medical and electronic apparatus, equipment, instruments and devices, namely, medical and electronic devices 150 1-n used in self-injectable medication procedures to collect waste materials;
- electronic medical apparatus, equipment, instruments and devices, namely; containers for medical waste;
- containers specially made for medical waste; boxes adapted for medical waste products;
- containers specially adapted for the disposal of medical instruments, syringes, and other contaminated medical waste;
- receptacles for applying the medicines, namely, cannulas for anaesthetics with receptacles;
- medical waste container, namely, sharps container for disposing of injectable medication;
- injection needles and syringes for medical use; drug delivery devices, namely, drug delivery systems;
- medical delivery apparatus, equipment, instruments, devices and systems for delivering medicaments, pharmaceuticals, vaccines and other substances intradermally, transdermally, subcutaneously or otherwise through the skin; electronic analysers for medical purposes, namely, skin moisture analysers for medical purposes, analysers for bacterial identification; electronic medical apparatus, equipment, instruments and devices for monitoring, detecting, measuring and recording data relating to the behaviour of humans taking medication;
- electronic monitoring instruments for medical use, namely, medical instruments for monitoring oximetry, gas analysis and vital signs; measuring apparatus, namely, electronic drop counters for medical use;
- inhalers for medical use; medical inhalers, namely, anaesthetic inhalers sold empty, hydrogen inhalers for medical use, sold empty; pumps for medical use to dispense pharmaceuticals from containers;
- medical apparatus, equipment, instruments and devices containing medicaments, pharmaceuticals, vaccines and drugs, namely, automatic vaccination apparatus, electromagnetic drug delivery apparatus for medical use, injection device for pharmaceuticals;
- medical delivery apparatus, equipment, instruments, devices and systems containing medicaments, pharmaceuticals, vaccines and drugs for delivery thereof intradermally, transdermally, subcutaneously or otherwise through the skin; parts and fittings for all the goods mentioned above. Unused medications or packaging for therapeutics, treatments or drugs.

According to some embodiments of the invention, the pathogen care item may be a vaccine syringe. The item identification unit 123 may be configured to detect a state of the syringe received in the storage container 123 of the PC device 120. The state of the syringe may indicate whether the vaccine has been properly or validly administered. For example, a fully compressed syringe may indicate that the syringe is empty, thus indicating that the full dose of the vaccine has been administered. The compression level may be detected based on the position of the syringe piston in the syringe tube. The information related to the detected state of the pathogen care item may be added to the PC data record, which is transmitted to the PC platform for further processing and validation. For example, the state of the pathogen care item may be used by the PC platform 110 to verify that the pathogen care event was validly performed. For example, if the identification unity 122 detects that the syringe is in a compressed state, processor 114 may consider that the vaccine has been validly administered. Otherwise, the processor may indicate an invalid administration of the vaccine or another related pathogen care item, e.g. therapeutic drug.

The PC device 120 may be configured for transmitting the generated pathogen care (PC) data record to the PC platform based associated via a communication module 125 such as a known cellular/wireless module. The identification unit 123 may comprise a processing unit in communication with a timer configured to timestamp the pathogen care items deposited in the storage container 123. The timestamped information may be transmitted as part of the pathogen care data record to the PC platform for storage in the database module 112 and processing by the processing unit 114.

The system 100 may comprise a Pathogen Care Management platform 110. The PCM platform 110 may be configured for receiving, via a communication link, the PC data records collected from one or more PC devices 120, e.g. during retrieval and/or disposal of the vaccine vial and/or identification information collected and transmitted from the electronic device 150 1-n.

The PCM platform 110 may comprise a database storage module 112 configured for storing data records associated with registered users. The data storage module 112 may be a computer database and/or a decentralised database, e.g. blockchain. For example, the database storage module 112 may be configured to store the digital health data records of registered patients. The health data records may include a range of information relating to the individual patients, such as medical information, personal information, identification credentials, and the like. The PCM platform 110 may further store digital data records associated with administration users, e.g. the practitioners administering the vaccine, which may contain similar information with the ones associated with the patient digital data records.

The PCM platform 110 may be running on a cloud platform, thus allowing each patient to access and manage their corresponding digital data records via a pathogen care software application. The software application may be downloaded and operated on an electronic device 150 1-n associated with a patient scheduled to attend the pathogen care event, a healthcare professional (HCP), or another authorised user. The pathogen care software application may enable users, depending on their credentials, to access and manage their digital health data records, update information, receive and access information regarding a pathogen care event, e.g. vaccination schedule, access vaccination aid and assistance resources, review vaccination history and status, modify vaccination schedule, consent to the vaccination schedule, upload their vaccine certificates to their electronic wallet or other Smartphone enabled app for easy access and the like. Furthermore, the pathogen care software application may be used to authenticate users for operating the PC device 120, as previously described. The software application may be configured to generate for each individual patient a pathogen care event schedule and plan based on certain information obtained directly from the patient and/or patient's health record. For example, the pathogen care event schedule may be based on the patient's health status, medical history, vaccination history, recommendation and/or policies of an authorised health authority etc. The pathogen care software application may be further configured to provide notifications and reminders to assist each patient in being timely and effectively adhere to a scheduled pathogen care event, e.g. a vaccination event.

The PCM platform 110 further comprises a processing unit 114 configured for processing the information received from the PC device 120, e.g. the PC data record and/or the identification data received from the electronic device 150 1-n. The processing unit 114 may be configured to verifying the validity of the vaccine administration event based on the comparison of the information received from the VAV devices 120 1-n and/or electronic devices 150 1-n with the information stored in the digital health data records stored in the VAV registry. For example, processing unit 114 may compare the type of vaccine administered to the individual patient with the expected vaccine information stored in the digital health data record. The processing unit 114 may be configured to update the digital data health record of each patient with information received from the PC device 120. For example, in response to a valid verification of the vaccination event, i.e. the vaccination of the individual was successful, the processing unit may be configured to update the vaccination status of the patient in the corresponding digital health data record. Furthermore, processing unit 114 may update the health status of the patient according to the information contained in the corresponding digital health record. Furthermore, the processing unit 114 may be configured to assign a confidence/risk score to the health status of the patient. For example, the processing unit, based on the physical location of the patient, which may be shared via the software application, may adjust the confidence score of the health status of the patient, indicating whether, for example, a healthy patient is likely to become sick based on for example. For example, in the case where the patient travels to a country with a high level of infectious diseases, then the confidence score of the health status of the patient may be lowered. As such, a corresponding action may be issued by the PCM platform directly or from a third party, such as that the patient needs to stay in quarantine for a number of days, a vaccine/drug needs to be administered, a test sample needs to be taken, schedule a visit to the doctor and the like.

The PCM platform 110 may comprise a certification module 1143 configured to generate a medical certificate to the patient, in response, for example, to a valid pathogen care event being detected, e.g. verification of a valid vaccination. The certificate issued may be sent to the user and uploaded to a desirable third party health application.

As shown in Figure 1A, the PCM platform 110 may be linked to a centralised certification system 130 of a health authority before the issuance of certificates and/or registration of issued certificates with an authorised health authority. The issued certificate may be linked to the digital health record of the patient. The PCM platform 110 may be further linked to a supply chain system 140 configured to manage the supply and/or production of pathogen care items. For example, the PCM platform 110 may provide information to the supply system of a manufacturer/distributor of the pathogen care item and accordingly place an order for the delivery of a new pathogen care item via an order management system. The supply chain system 140 may comprise an order management system configured for receiving pathogen care item orders from the PCM platform 120

According to embodiments of the present disclosure, the PCM platform 110 may be communicatively coupled to a pathogen care item storage device 170 configured for storing pathogen care items associated with the pathogen care event. The PC item storage device 170 may be part of the pathogen care management system 100 and configured to be operated by the PC platform 110 for the dispensation of pathogen care items. For example, the PC platform 110 may control, based on the information received from an electronic device 150 1-n associated with an administrator and/or the PC device 120, dispensation of a pathogen care item. The PC platform 110 may further monitor a set of parameters associated with the PC item storage system 170 such as temperature, inventory of pathogen care items, the time elapsed between a dispensation of a pathogen care item and detection of one or more deposits of corresponding pathogen care item in the PC device 120.

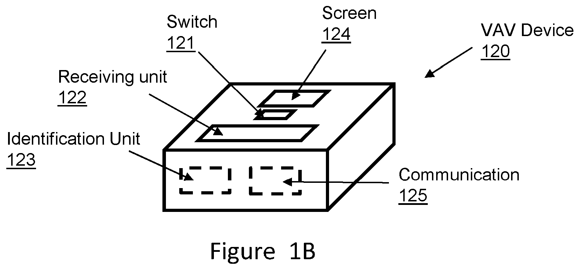

An exemplified pathogen care device 120 is shown in Figure 1B. The PC device 120 comprises, for example, a storage container 122 configured for storing pathogen care items disposed of through the receiving unit 122. The storage container, also referred to as the storage bin/compartment, is removable which can be replaced when full. The receiving unit 122 may be a module comprising an opening for receiving the pathogen care item, e.g. a sharp, which is then may be directed to the storage container. The PC device 120, also referred to as Vaccine Administration Verification (VAV) device, may be optionally provided with an operating switch 121 to indicate the state of the device, e.g. switch on or off, and a screen 124. When a user deposits a pathogen care item into the receiving unit 121, the disposal or deposit may trigger the operation of the item identification unit 122. The identification unit 122 may comprise one or more sensors configured to detect, recognise and/or identify received Pathogen care items associated with a pathogen care event, e.g. an injection of a specific vaccine. According to some embodiments, the identification unit 122 may be configured to analyse an image obtained from the detected pathogen care item. The identification unit 122 may be configured for scanning a one-dimensional or two-dimensional identification code attached to the pathogen care item, as previously described. For example, the pathogen care item identification unit 122 may comprise an RFID reader configured to read information from an RFID tag attached to the VAV item.

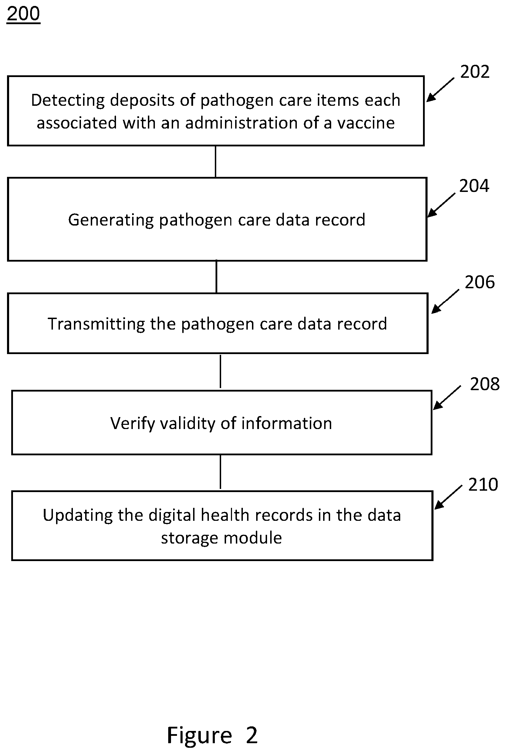

An exemplified method 200 for tracking and managing pathogen care events is shown in Figure 2. According to some embodiments of the disclosure, the PC device detects 202 one or more deposits of pathogen care items, e.g. vaccine sharps deposits. Each of the pathogen care items may be associated with an administration of an injectable medicament, e.g. a vaccine. Based on the detected pathogen care items, at step 204, the PC device 120 generates a pathogen care data record. The PC data record may comprise information associated with the deposit of the pathogen care item, e.g. type of vaccine, date and time, a pathogen care item identification data, e.g. photo, electronic device connected to the PC device 120, and the like. The generated data record is transmitted, at step 206, to the PCM platform 110, where it is processed at step 208 by the processing unit 114. The processing unit 114 may be configured for updating at step 210 the information in the patient's digital health data record with the information contained at least in the PC data record and/or a user identification data record received from an electronic device 150 1-n. The processing unit 114 is configured to verify the validity of the pathogen care event. For example, the processing unit 114 may be configured to compare information received from the PC device 120 and/or an electronic device 150 1-n with reference information stored in the database, e.g. information stored in a user digital health record. In the case of a valid pathogen care event, the processor is configured for updating pathogen care information in the corresponding digital health data record associated with the pathogen care event, e.g. vaccine administration information such as date, batch number and the like.

It should be noted that the processing unit 114 may update the digital health record of the patient with information from the PC data record irrespective of the validity of the vaccine administration event. For example, in the case of rejection, the pathogen care information of the health record may be updated with the rejected notification, and a set of follow-up procedures may be initiated, e.g. schedule a new appointment, follow-up with a doctor, and the like.

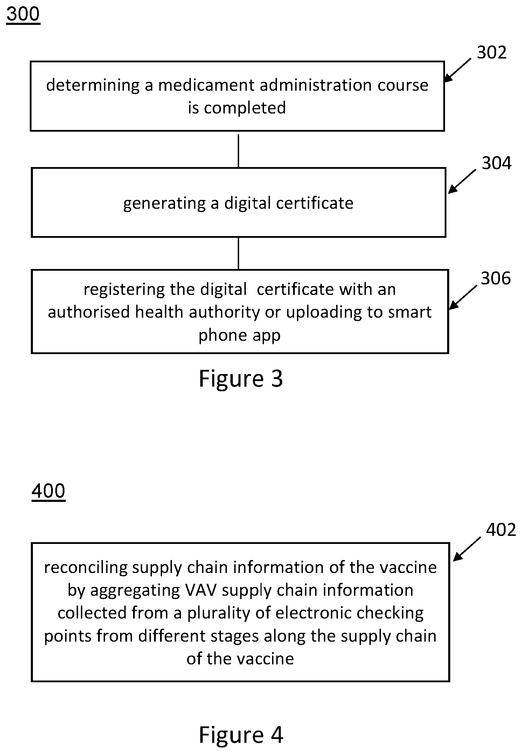

As shown in figure 3, step 210 of updating the digital health record may comprise a series of additional steps. For example, at step 301, the processing unit 114 may verify the validity of the pathogen care event by comparing the information received from the PC device 120 and/or electronic device 150 with the information stored in the digital data record of the patient or another location in the database. At step 302, the processing unit 114 may be configured to evaluate based on information in the digital health data record whether a prescribed pathogen care event course has been completed, e.g. vaccination event. In step 304, a medical certificate may be issued following a valid and completed pathogen care event course. The certificate may be registered with an authorised authority, e.g. a governmental authority, and may further be associated with the ID of the patient, e.g. biometric ID, at step 306. For example, the certificate may be transmitted to a computer device 150 associated with the individual. The certificate may also be available for download or viewing via the pathogen care software application or via a smartphone app. This certificate may be issued as a softcopy, in a software application, or a hardcopy, on paper. The patient may, for example, use the certificate at border control, e.g. a port or an airport, where based on the presentation of the biometric features of the patient, a scanner may retrieve an individual's validated digital certificate allowing entry into that territory.

As previously discussed, the PC device 120 may be configured to communicate with storage module 170 for the dispensation of pathogen care items, e.g. vaccine vials. As such, the PC device 120 may be configured to register the pathogen care items dispensed and accordingly determine, based on information obtained during the detection of a pathogen care item deposit, if the correct corresponding pathogen care item has been deposited. For example, in the case of a vaccine vial being dispensed, the processing unit 114 would expect that within a predetermined amount of time would receive information from the PC device 120 of the detection of one or more corresponding vaccine sharps. In this way, the system 100 of the present invention enables tracking of the pathogen care item. For example, certain vaccines may need to be used within a certain time frame; otherwise, their effectiveness may be compromised. Furthermore, in this way, misappropriate, and counterfeiting of the pathogen care items may be prevented. SHELF LIFE discussed in more detail above - perhaps its sits here.

The PCM platform 110 may issue one or more notifications to at least one authorised user of the PCM system 100. For example, the PCM platform 110 may issue a notification indicating any one of or combiation thereof: the health status of an individual patient, provide information on the time frame between the dispensation of a pathogen care item and detection at the PC device 120, whether the pathogen care item was administered within the specified time frame, the number of unused pathogen care item stored in the, issue a pathogen care item stock notification indicating the number of pathogen care items remaining/used and whether a new pathogen care item order needs to be placed, and the like.

The PCM platform 110 may reconcile at step 402 supply chain information for the supply and delivery of pathogen care items by aggregating information collected from a plurality of electronic checking points located at different stages along the supply chain journey of the pathogen care items. The supply chain information may comprise information collected during manufacturing, and/or transportation, and/or delivery, and/or administration. An authorised user may re-order the vaccine through the software application. In addition, the PCM platform 120-may automatically place an order for pathogen care item to the order management system of the supply chain system 140, based on information collected from any one of or combination of PC item storage module 170, electronic device 150 1-n, and/or PC device 120.

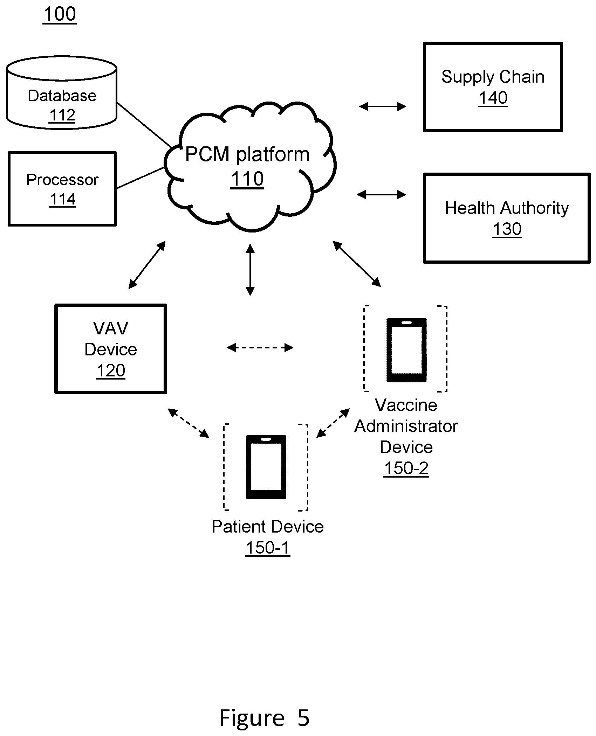

An example of a Pathogen care management system 100 used in monitoring and managing pathogen care events according to embodiments of the present disclosure is shown in Figure 5. The PC system 100 of figure 5 may be used for monitoring and verifying the occurrence of a pathogen care event, which may involve the administration of a vaccine or another medicament to a patient according to a prescribed pathogen care course. The PCM system 100 may be provided with at least one PC device 120 configured to detect sharps deposits of spend pathogen care item, e.g. vaccine syringes, and accordingly generate for each sharp deposit a PC data record comprising information associated with the pathogen care event. As previously mentioned with reference to figure 1, the PC device 120 may be communicatively coupled to at least one first electronic device 150-1 configured to run a PC software application configured to identify and/or register a patient prior to the pathogen event. The PC software application, as previously indicated, may be operated by a patient to access and manage their unique health data record, as well as completing any necessary documentation prior to the pathogen care event, e.g. consent forms, and the like. The health care professional (HPC) administering the pathogen care item, e.g. the vaccine, may be provided with a second electronic device 150-2 running a corresponding software application associated with the PCM system 100. At least one of the first and/or second electronic devices 150-1 and 150-2 may be communicatively coupled to the PC device 120 via a wireless communication network and/or a short-range wireless communication channel, e.g. Bluetooth, Near Field Communication (NFC) and the like. For example, the electronic device of the patients and/or the vaccine administrator may be connected with the PC device 120 using a Bluetooth connection. A software application may be running on the PC device 120 and electronic devices 150 1-2 that may initiate the connection/pairing of the device, e.g. by alerting the user when the PC device 120 is within a connection range and accordingly prompting the user to connect to the PC device 120. The information generated by the PC device during the pathogen care administration event is transmitted to the PCM Platform 110. As previously indicated, the information received is processed and collated/compared with information stored in the digital health record of the patient to determine the validity of the pathogen care administration event.

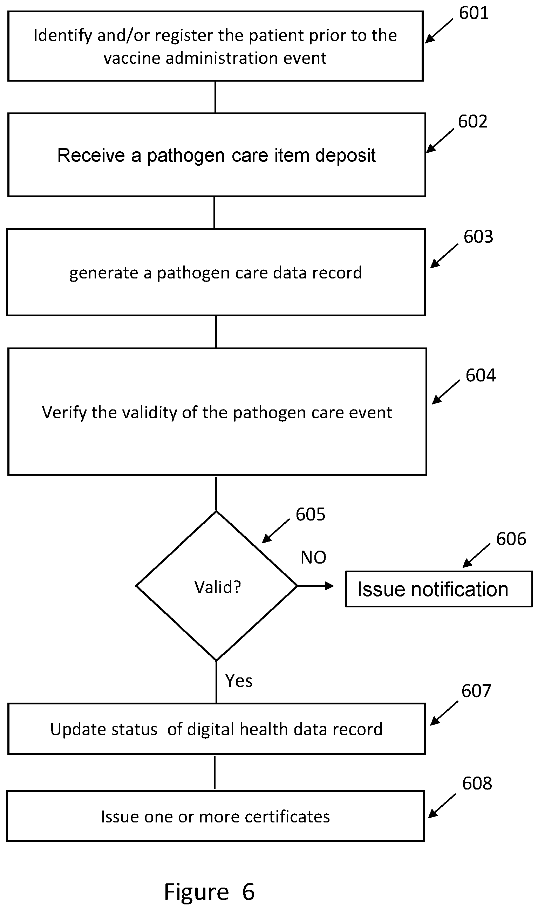

Figure 6 shows an example of a process flow for administering a pathogen care item to a patient at a healthcare facility, e.g. a clinic, a pharmacy, and the like. It should be noted that although the process flow has been exemplified using as example a vaccine administration, the method can be equally applied to any other medical and/or clinical event such as drug trials, administration of a sediment, test sample collection, and the like. Prior to the medical event, e.g. a vaccine administration, at step 601, the patient downloads the pathogen care software application on his/her electronic device 150, such as a mobile phone. The individual patient may register or create a profile in the PCM platform110 and completes the necessary documentation, such as providing consent. At step 601, the patient identity may also be confirmed using biometric authentication, e.g. a fingerprint obtained from a biometric sensor of the electronic device or similar. The patient may further use the software application to request vaccination appointment on his/her own initiative or according to vaccination policies of the relevant health authorities.

When the patient attends the vaccine clinic, a health care professional may identify the user based on their biometric features or in another way, e.g. identification document, and the like. Once the patient is identified, the patient health data record may be retrieved from the PC platform 110through an administrator software application. A photo of the individual and/or a photo of the individual's ID may be taken. The photo may be uploaded to the database through the administrator application. The healthcare professional may request through the PC platform 110 the dispensation of the pathogen care item from a storage system 170. Similarly, the Health care professional (HCP) may retrieve the vaccine from the storage system 170 manually. In any case, the dispensed vaccine may then be scanned, or the Identification number may be manually entered on the administration software application. The information associated with the dispensed vaccine may be validated by the PCM platform 100, and a notification may be issued to the software application operated by the healthcare professional. The healthcare professional administers the vaccine, and the spend sharp is deposited into the PC device 120 as previously described. The PC device 120 detects, at step 602, the vaccine sharp deposit and generates, at step 603, a PC data record containing a range of information, e.g. vaccine type, a vaccine image, timestamping information, proximity detection information, and the like. The PC data record, along with other information (e.g. identification data generated from a connected electronic device 150), are transmitted to the PCM platform 110 along with the user identification data record generated by the administrator software application during identification of the patient. The PC platform 110 verifies the validity of the vaccine administration event at step 604, as previously discussed. In response to a valid vaccination, the vaccine status of the digital health record is updated at step 607. In the case where the vaccine administration is deemed invalid or, further confirmation is needed, a notification may be issued to a corresponding user, e.g. healthcare professional, patient, doctor, health authority, and the like, at step 606. At step 608, a vaccine certificate may be generated for a valid vaccine administration event, which is associated with the corresponding digital health record of the patient. A confirmation of the vaccine administration may be sent to both the patient attending the vaccination and the healthcare professional through their respective software applications. The vaccine certificate may also contain a photo of the individual taken at the vaccine event as additional verification. The PC platform 110, based on the information received during the pathogen care event, may determine the number of available vaccines and accordingly place an order in the order management system of the supply chain system 140. The vaccine may be re-ordered to ensure a sufficient supply of vaccines for future administration.

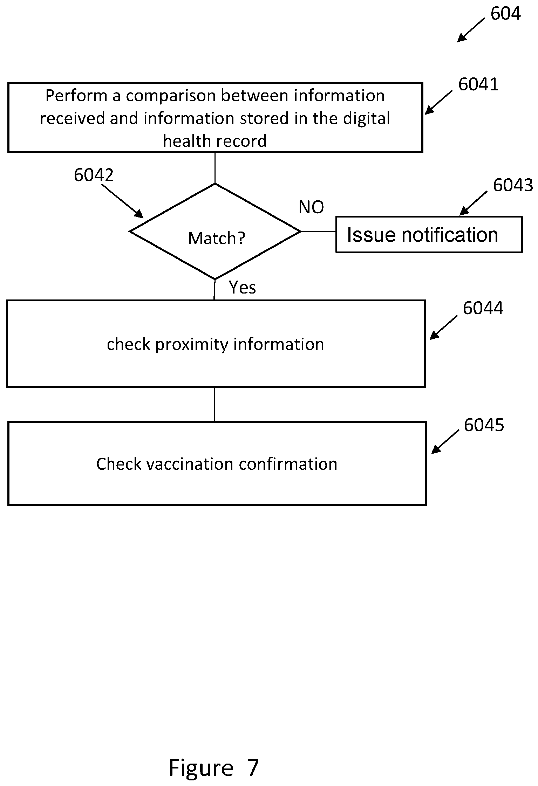

Figure 7 shows an exemplified method for verifying the validity of the vaccine administration at step 604 of figure 6. At step 6041, the processing unit may compare the information received from the PC device 120 and/or a connected electronic device associated with an administrator user and/or patient with the information contained in the digital health data record. For example, the processing unit 114 may determine based on the comparison that the correct vaccine and/or vaccine dose was given to the patient. If there is a match between the information compared in step 6041, then a notification is issued at step 6403. For example, a notification may be issued to the patient and/or vaccine administrator via the pathogen care software application. An optional additional verification step may be performed at step 6402, whereby the processing unit may use the information received from the PC device 120 and/or an electronic devices 150 1-n associated with the HPC user, i.e. the administrator, and/or the patient electronic device to verify that the patient was present at the pathogen care event. For example, the verification may be based on information associated with the proximity of the patient's electronic device to the PC device 120 and/or the electronic device of the administrator.

Furthermore, the patient's presence may be verified based on the scanning of an identification code, e.g. a QR code, and/or based on biometric scanning, as previously discussed. Furthermore, the presence of the patient may be verified by the administrator manually. Further notification may be issued to the administrator of the pathogen care event requesting to confirm the pathogen care event and the presence of the patient at step 6044. The processing unit 114 may further request a vaccine confirmation from the vaccine administrator to confirm that the vaccine was administered according to a predetermined set of standards associated with the prescribed vaccination course.

Although the above process is described in a context of a clinical setting for vaccine administration, it is understood that the setting for administration could be a hospital, clinic or home setting. The vaccine may be administered by a nurse in a clinic or self-administered by the individual.

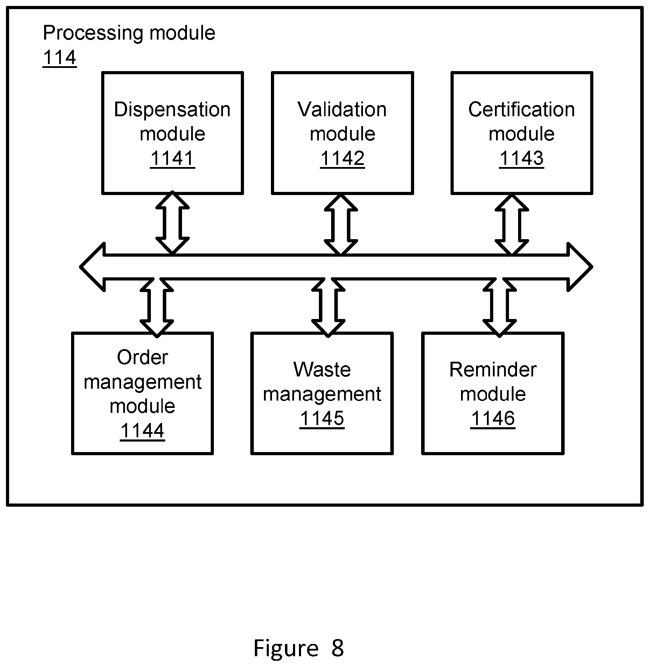

Figure 8 shows an exemplified architecture of the processing unit 114 according to embodiments of the present disclosure. As previously discussed, the processing unit may be provided with a validation module 1141 configured to verify the validity of the pathogen care event based on information received from one or more electronic devices 120 1-n and/or the PC device 120, as previously discussed. The processing unit 114 may be provided with a dispensation module 1142 configured to monitor and manage the dispensation of pathogen care items from the storage module 170, and further monitor a set of parameters associated with the PC item storage system 170 such as temperature, the elapsed time between dispensation and use of a pathogen care item, inventory, and the like. A certification module 1143 may be provided to issued certificates in response to a valid pathogen care event. For example, the certificates may be in digital form and can be uploaded in various software applications. The certificates may also be printed and so on. The issued certificates may be stored in a distributed ledger system. An order management module 1144 may be provided to monitor the supply of pathogen care items to a location, e.g. a vaccination centre. The order management module 1144 is communicatively coupled to the order management system of the supply chain system 140, as previously discussed. Then processing module may be provided with a waste management module 1145, which is connected with a waste management system 160 for the collection and processing of the pathogen care items disposed of in the storage bin of the PC device 120. A reminder module 1146 may further be provided for issuing reminders and/or notifications to the patient, the health care professional, and/or another registered user. The reminder module may send notification and reminders to one or more registered user as previously described. The frequency and number of notifications may be adjusted in response to the urgency of the event.

In general, the present disclosure offers an effective solution to improve adherence of patients to a prescribed medication and/or clinical schedule such as a vaccination course, test sample collection, drug trial, and the like. The processing unit 114 of the PCM platform 110 is configured to monitor adherence levels of an individual patient and accordingly issue, via the reminder module 1146, a set of reminders triggered based on the behaviour of the patient. The timing at which the reminders are triggered may be dynamically adjusted by the reminder module 1146 based on information associated with the patient e.g. timing of historical deposits of pathogen care items, calendar schedule of the user, location of the patient and proximity to the PC device 120, and the like. In this way, the medicament adherence of a patient may be improved via timely interventions. If necessary, the reminder module 1146 may initiate other forms of interventions to prompt the patient to take a prescribed medication. For example, manual interventions may be requested by the reminder module 114 by sending notifications to a healthcare professional, or another designation person e.g. relative. The present invention offers an effective solution for supporting patients achieve a greater medication adherence. For example, data collected by the PC platform 110 e.g. detected medical items deposited in a PC device 120, may provide insights into patient behaviour to allow analysis of patterns within individual patients, patient groups, geographical populations, prescribed medicament, and other type of information. The medical management system 100 of the present invention may comprise an ensemble predictive model to improve the accuracy of predictions made on patient medicament adherence behaviours, thus enabling more accurate and timely notifications to be issued resulting an improvement of the patient adherence performance.

The system of the present disclosure may further help patients to manage complex medication regimes involving one or more medicament through the generation of timely prompts and reminders. In this way, the system of the present disclosure simplifies the administration process involved in a medicament schedule while providing validated data on the medicament administration behaviour of a patients to health care professional. The ability to record the time of disposal of a pathogen care item in the PC device 120 and reconcile the information back to the patient's medication schedule in the PC platform 110, enables the calculation of an adherence score that may be used to inform patients on their adherence performance and alert the healthcare providers. The adherence score may be used to calculate the health status of the patient as previously described. The medication schedule of the patient may be stored in the corresponding digital health record, which may be updated as previously described.

As previously described, once the patient has successfully completed a medicament course, e.g. one or more vaccine shots to achieve immunity against a specific pathogen or disease, a digital vaccine certificate may be generated 304 and issued to the patient through the patient application. The certificate may be registered 306 with the corresponding health authority. The patient may download or retrieve the vaccine certificate when needed. For example, relevant authorities may require an individual to show a specific vaccine certificate in order to allow the individual to travel or enter a premise. The vaccine certificate may be signed or encrypted such that the authority can verify the validity of the vaccine certificate to ensure authenticity. The certificate may have an expiry date depending on the specific type of vaccine, i.e. whether the vaccine is life-long, effective for a specific number of years, or seasonal. The PCM system 100 of the present disclosure may also be used for tracking and monitoring a clinical/medical diagnostic test, for example, collecting of a swab for detection of Covid-19 by Polymerase Chain Reaction or detection of antibody or antigen.

For example, the following steps may be involved:
- a screening test is scheduled, and the patient is notified via the software application or requests a test through the app;
- the patient attends the event at a clinic/pharmacy/ testing centre and identifies through an electronic device 150 1-n using his/her biometric ID or through the app registration system.
- a blood sample, swab or another sample taken;
- the sample is sealed and dropped into the storage container of the PC device 120.
- PC data record is generated, which is transmitted to the PCM platform for storage and processing.
- The storage container (or Device 120) may be configured to keep the medical test sample cold;
- the sample is removed from the storage box for processing in the medical lab
- The lab may be connected to the PCM platform to determine the required diagnostic analysis on the sample
- Sample is processed, and the results are transmitted to the PCM platform;
- patient and/or doctor receives the notification of the results;
- the PCM platform updates the health status of the patient and assigns a confidence score;
- patient is notified and may follow up with a doctor.
- A certificate of results may be issued that can be uploaded to the patients' smartphone app.

## Claims

1. A pathogen care management system (100) for managing and tracking pathogen care vaccine administration events associated with a patient user, the system (100) comprising:
a pathogen care software application running on at least one administrator electronic device (150 1-n) associated with an administrator of the pathogen care event configured to identify and register a user prior to a pathogen care event, the pathogen care software application configured to generate, during operation, a user identification data record comprising identification information associated with the user and confirmation of proximity of a patient electronic device associated with the patient user during the pathogen care event;
at least one pathogen care sharps bin device (120 1-n) communicatively coupled to the at least one administrator electronic device (150 1-n), the sharps bin device (120 1-n) configured to detect:
(i) deposits of at least one pathogen care item associated with the pathogen care vaccine administration event;
(ii) proximity of the patient electronic device thereto during the vaccine administration event; wherein in response to detecting a pathogen care item being placed in a storage compartment of the sharps bin device (120 1-n), the at least one sharps bin device (120 1-n) is configured to generate a pathogen care data record associated with the patient user identified by the pathogen care software application including data verifying (i) the deposition of the vaccine administration item and (ii) the proximity of the patient electronic device;
a pathogen care management, PCM, platform (112) communicatively coupled to the at least one sharps bin device (120 1-n) and the
at least one administrator electronic device (150 1-n) for receiving corresponding pathogen care data records and user identification data records, the PCM platform (112) comprising:
a data storage module (140) storing a plurality of digital health records associated with users, each digital health record comprising pathogen care data including: the pathogen care item deposit data, the patient electronic device proximity data, and patient identification data associated with a unique patient user, and
a processing unit (114) configured to update the digital health record associated with the identified patient user with information received from the sharps bin device (120 1-n) and/or at least one electronic device (150 1-n).

2. The pathogen care management system (100) according to claim 1, wherein the processing unit (114) of the PCM platform (110) comprises a validation module (1142) configured, prior to updating a digital health record, to validate the corresponding pathogen care event by comparing information contained in the pathogen care data record data and/or patient user identification data record with reference information retrieved from a database of the data storage module (112) and/or stored in the digital health record of the corresponding patient user, wherein verifying the validity of the pathogen care event further comprises checking (6044) the proximity information and checking (6045) vaccination confirmation.

3. The pathogen care management system (100) according to claim 2, wherein in response to a valid pathogen care event, the processing module (114) is configured to update a health status of the patient user digital health record and issue a corresponding certificate, which is stored in the digital health data record of the patient user and further transmitted to a patient user software application running on a patient user electronic device (150 1-n) associated with the patient user.

4. The pathogen care management system (100) according to claim 2 or 3, wherein in response to an invalid pathogen care event, the processing module (114) is configured to update the health status of the corresponding digital health record with the rejection notification.

5. The pathogen care management system (100) according to any one of the preceding claim, wherein the processing unit (114) comprises a reminder module (1146) configured to issue one or more reminders associated with a pathogen care event, based on information contained in the corresponding user digital health records and/or information received from an external system, the reminders being transmitted to one or more electronic devices (150 1-n) associated with a patient user at a selected frequency.

6. The pathogen care management system (100) according to claim 5, wherein the reminder module (1146) is configured to adjust the reminder transmission frequency based on a classification of the reminders into normal, urgent, and emergency types.

7. The pathogen care management system (100) of any one of the preceding claims, wherein the pathogen care data record comprises information obtained from an image of the pathogen care item and/or scanning of an identification code associated with the pathogen care item.

8. The pathogen care management system (100) of any one of the preceding claims, wherein the pathogen care software application is configured to identify a patient user based on a biometric ID of the patient user comprising at least one biometric feature associated with the patient user.

9. The pathogen care management system (100) of any one of the preceding claims, wherein the pathogen care software application is configured to identify a user based on scanning of an identification code presented on a screen of an electronic device associated with the patient user.

10. The pathogen care management system (100) of any one of the preceding claims, wherein the processing unit (114) comprises a dispensation management (1141) module configured to activate dispensation of a pathogen care item from a storage receptacle (170), based on information contained in at least the patient user identification data record.

11. The pathogen care management system (100) of claim 10, wherein the dispensation management module (1141) is configured to monitor a set of parameters of the storage receptacle (170).

12. The pathogen care management system (100) of any one of the preceding claims, wherein the sharps bin comprises:
a removable compartment configured to receive pathogen care item deposits;
at least one sensor module (123) configured to detect the pathogen care item deposit;
a timestamping module configured to timestamp the pathogen care item deposit;
a processing module for generating the pathogen care data record; and
a communication module (125) configured for transmitting the pathogen care data record to the PCM platform (110).

13. A method for recording and validating pathogen care vaccine administration events associated with patient users, the method comprising:
registering (601), at an administrator electronic device (150 1-n) running a pathogen care software application, identification information associated with a patient user prior to a pathogen care event:
generating, by the electronic device (150 1-n), a user identification data record comprising identification information associated with the identified user and confirmation of proximity of a patient electronic device associated with the patient user during the pathogen care event;
detecting, at a pathogen care sharps bin device (120 1-n) communicatively coupled to the administrator electronic device, a deposit (602) of a pathogen care item associated with the pathogen care vaccine administration event being placed at a compartment of the pathogen care sharps bin device;
detecting at the pathogen care sharps bin device (120 1-n) proximity information confirming the proximity of the patient electronic device thereto during the vaccine administration event;
generating (603), at the pathogen care device (120 1-n), a pathogen care data record associated with the identified user including data verifying (i) the deposition of the pathogen care vaccine administration item associated with the pathogen care event being placed at a compartment of the pathogen care sharps bin device and (ii) the proximity of the patient electronic device at the pathogen care event;
receiving, at a pathogen care management, PCM, platform (110), the pathogen care data record and the user identification data record;
verifying (604), by a processing unit (114) of the PCM platform (110), the validity of the information received by comparing information contained in the pathogen care data record and/or the user identification data record with reference information, wherein verifying the validity of the pathogen care event further comprises checking (6044) the proximity information and checking (6045) vaccination confirmation; and
updating (607), by the processing unit (114) based on the validity of the information, received, a digital health record associated with the user.

14. The pathogen care management system (100) according to claim 1, comprising a vaccine administration verification, VAV, system, wherein the pathogen care item comprises a vaccine administration item and the pathogen care event comprises a vaccine administration event.

15. The pathogen care management system (100) according to claim 1, wherein the pathogen care software application is configured to confirm the proximity of the patient's electronic device including for example by scanning of an identification code presented at the screen of the electronic device associate with a user or pairing through a short-range wireless connection.

## Patentansprüche

1. Pathogen-Pflege-Verwaltungssystem (100) zum Verwalten und Verfolgen von Pathogen-Pflege-Impfstoff-Verabreichungsereignissen, die mit einem Patienten-Benutzer assoziiert sind, wobei das System (100) Folgendes beinhaltet:
eine Pathogen-Pflege-Softwareanwendung, die auf mindestens einer elektronischen Administratorvorrichtung (150 1-n) läuft, die mit einem Administrator des Pathogen-Pflege-Ereignisses assoziiert ist, und konfiguriert ist, um einen Benutzer vor einem Pathogen-Pflege-Ereignis zu identifizieren und zu registrieren, wobei die Pathogen-Pflege-Softwareanwendung konfiguriert ist, um während des Betriebs ein Benutzeridentifikationsdatenverzeichnis zu erzeugen, das Identifikationsinformationen, die mit dem Benutzer assoziiert sind, und eine Bestätigung der Nähe einer elektronischen Patientenvorrichtung, die mit dem Patienten-Benutzer assoziiert ist, während des Pathogen-Pflege-Ereignisses beinhaltet;
mindestens eine Pathogen-Pflege-Kanülensammelbehältervorrichtung (120 1-n), die kommunikativ mit der mindestens einen elektronischen Administratorvorrichtung (150 1-n) gekoppelt ist, wobei die Kanülensammelbehältervorrichtung (120 1-n) konfiguriert ist, um Folgendes zu detektieren:
(i) Ablagerungen von mindestens einem Pathogen-Pflege-Artikel, der mit dem Pathogen-Pflege-Impfstoff-Verabreichungsereignis assoziiert ist;
(ii) Nähe der elektronischen Patientenvorrichtung dazu während des Impfstoff-Verabreichungsereignisses:
wobei als Reaktion auf das Detektieren, dass ein Pathogen-Pflege-Artikel in einem Aufbewahrungsfach der Kanülensammelbehältervorrichtung (120 1-n) platziert ist, die mindestens eine Kanülensammelbehältervorrichtung (120 1-n) konfiguriert ist, um ein Pathogen-Pflege-Datenverzeichnis zu erzeugen, das mit dem Patienten-Benutzer assoziiert ist, der durch die Pathogen-Pflege-Softwareanwendung identifiziert wird, umfassend Daten, die (i) die Ablagerung des Impfstoff-Verabreichungsartikels und (ii) die Nähe der elektronischen Patientenvorrichtung verifizieren;
eine Pathogen-Pflege-Verwaltungs-Plattform, PCM-Plattform, (112), die kommunikativ mit der mindestens einen Kanülensammelbehältervorrichtung (120 1-n) und der mindestens einen elektronischen Administratorvorrichtung (150 1-n) gekoppelt ist, um entsprechende Pathogen-Pflege-Datenverzeichnisse und
Benutzeridentifikationsdatenverzeichnisse zu empfangen, wobei die PCM-Plattform (112) Folgendes beinhaltet:
ein Datenspeichermodul (140), das eine Vielzahl von digitalen Gesundheitsverzeichnissen speichert, die mit Benutzern assoziiert sind, wobei jedes digitale Gesundheitsverzeichnis Pathogen-Pflege-Daten beinhaltet, die Folgendes umfassen: die Pathogen-Pflege-Artikel-Ablagerungsdaten, die Näherungsdaten der elektronischen Patientenvorrichtung und Patientenidentifikationsdaten, die mit einem eindeutigen Patienten-Benutzer assoziiert sind, und
eine Verarbeitungseinheit (114), die konfiguriert ist, um das digitale Gesundheitsverzeichnis, das mit dem identifizierten Patienten-Benutzer assoziiert ist,
mit Informationen zu aktualisieren, die von der Kanülensammelbehältervorrichtung (120 1-n) und/oder mindestens einer elektronischen Vorrichtung (150 1-n) empfangen werden.

2. Pathogen-Pflege-Verwaltungssystem (100) gemäß Anspruch 1, wobei die Verarbeitungseinheit (114) der PCM-Plattform (110) ein Validierungsmodul (1142) beinhaltet, das konfiguriert ist, um vor dem Aktualisieren eines digitalen Gesundheitsverzeichnisses das entsprechende Pathogen-Pflege-Ereignis zu validieren, indem Informationen, die in den Pathogen-Pflege-Datenverzeichnisdaten und/oder dem Patientenbenutzer-Identifikationsdatenverzeichnis enthalten sind, mit Referenzinformationen verglichen werden, die aus einer Datenbank des Datenspeichermoduls (112) abgerufen und/oder in dem digitalen Gesundheitsverzeichnis des entsprechenden Patienten-Benutzers gespeichert werden, wobei das Verifizieren der Gültigkeit des Pathogen-Pflege-Ereignisses ferner das Prüfen (6044) der Näherungsinformationen und das Prüfen (6045) der Impfbestätigung beinhaltet.

3. Pathogen-Pflege-Verwaltungssystem (100) gemäß Anspruch 2, wobei als Reaktion auf ein gültiges Pathogen-Pflege-Ereignis das Verarbeitungsmodul (114) konfiguriert ist, um einen Gesundheitsstatus des digitalen Patientenbenutzer-Gesundheitsverzeichnisses zu aktualisieren und ein entsprechendes Zertifikat auszustellen, das in dem digitalen Gesundheitsdatenverzeichnis des Patienten-Benutzers gespeichert und ferner an eine Patientenbenutzer-Softwareanwendung übertragen wird, die auf einer elektronischen Patientenbenutzervorrichtung (150 1-n) läuft, die mit dem Patienten-Benutzer assoziiert ist.

4. Pathogen-Pflege-Verwaltungssystem (100) gemäß Anspruch 2 oder 3, wobei als Reaktion auf ein ungültiges Pathogen-Pflege-Ereignis das Verarbeitungsmodul (114) konfiguriert ist, um den Gesundheitsstatus des entsprechenden digitalen Gesundheitsverzeichnisses mit der Ablehnungsbenachrichtigung zu aktualisieren.

5. Pathogen-Pflege-Verwaltungssystem (100) gemäß einem der vorhergehenden Ansprüche, wobei die Verarbeitungseinheit (114) ein Erinnerungsmodul (1146) beinhaltet, das konfiguriert ist, um eine oder mehrere Erinnerungen auszustellen, die mit einem Pathogen-Pflege-Ereignis assoziiert sind, basierend auf Informationen, die in den entsprechenden digitalen Benutzergesundheitsverzeichnissen enthalten sind, und/oder Informationen, die von einem externen System empfangen werden, wobei die Erinnerungen an eine oder mehrere elektronische Vorrichtungen (150 1-n) mit einer ausgewählten Frequenz übertragen werden, die mit einem Patienten-Benutzer assoziiert sind.

6. Pathogen-Pflege-Verwaltungssystem (100) gemäß Anspruch 5, wobei das Erinnerungsmodul (1146) konfiguriert ist, um die Erinnerungsübertragungsfrequenz basierend auf einer Klassifizierung der Erinnerungen in normale, dringende und Notfalltypen anzupassen.

7. Pathogen-Pflege-Verwaltungssystem (100) gemäß einem der vorhergehenden Ansprüche, wobei das Pathogen-Pflege-Datenverzeichnis Informationen beinhaltet, die aus einem Bild des Pathogen-Pflege-Artikels und/oder Scannen eines Identifikationscodes, der mit dem Pathogen-Pflege-Artikel assoziiert ist, erhalten werden.

8. Pathogen-Pflege-Verwaltungssystem (100) gemäß einem der vorhergehenden Ansprüche, wobei die Pathogen-Pflege-Softwareanwendung konfiguriert ist, um einen Patienten-Benutzer basierend auf einer biometrischen ID des Patienten-Benutzers zu identifizieren, die mindestens ein biometrisches Merkmal beinhaltet, das mit dem Patienten-Benutzer assoziiert ist.

9. Pathogen-Pflege-Verwaltungssystem (100) gemäß einem der vorhergehenden Ansprüche, wobei die Pathogen-Pflege-Softwareanwendung konfiguriert ist, um einen Benutzer basierend auf Scannen eines Identifikationscodes zu identifizieren, der auf einem Bildschirm einer elektronischen Vorrichtung dargestellt wird, die mit dem Patienten-Benutzer assoziiert ist.

10. Pathogen-Pflege-Verwaltungssystem (100) gemäß einem der vorhergehenden Ansprüche, wobei die Verarbeitungseinheit (114) ein Abgabeverwaltungsmodul (1141) beinhaltet, das konfiguriert ist, um die Abgabe eines Pathogen-Pflege-Artikels aus einem Aufbewahrungsbehälter (170) basierend auf Informationen zu aktivieren, die in mindestens dem Patientenbenutzer-Identifikationsdatenverzeichnis enthalten sind.

11. Pathogen-Pflege-Verwaltungssystem (100) gemäß Anspruch 10, wobei das Abgabeverwaltungsmodul (1141) konfiguriert ist, um einen Satz von Parametern des Aufbewahrungsbehälters (170) zu überwachen.

12. Pathogen-Pflege-Verwaltungssystem (100) gemäß einem der vorhergehenden Ansprüche, wobei der Kanülensammelbehälter Folgendes beinhaltet:
ein entfernbares Fach, das konfiguriert ist, um Pathogen-Pflege-Artikel-Ablagerungen aufzunehmen;
mindestens ein Sensormodul (123), das konfiguriert ist, um die Pathogen-Pflege-Artikel-Ablagerung zu detektieren;
ein Zeitstempelmodul, das konfiguriert ist, um die Pathogen-Pflege-Artikel-Ablagerung zeitzustempeln;
ein Verarbeitungsmodul zum Erzeugen des Pathogen-Pflege-Datenverzeichnisses; und
ein Kommunikationsmodul (125), das konfiguriert ist, um das Pathogen-Pflege-Datenverzeichnis an die PCM-Plattform (110) zu übertragen.

13. Ein Verfahren zum Aufzeichnen und Validieren von Pathogen-Pflege-Impfstoff-Verabreichungsereignissen, die mit Patienten-Benutzern assoziiert sind, wobei das Verfahren Folgendes beinhaltet:
Registrieren (601), an einer elektronischen Administratorvorrichtung (150 1-n), auf der eine Pathogen-Pflege-Softwareanwendung läuft, von Identifikationsinformationen, die mit einem Patienten-Benutzer assoziiert sind, vor einem Pathogen-Pflege-Ereignis;
Erzeugen, durch die elektronische Vorrichtung (150 1-n), eines Benutzeridentifikationsdatenverzeichnisses, das Identifikationsinformationen, die mit dem identifizierten Benutzer assoziiert sind, und eine Bestätigung der Nähe einer elektronischen Patientenvorrichtung, die mit dem Patienten-Benutzer assoziiert ist,
während des Pathogen-Pflege-Ereignisses beinhaltet;
Detektieren, an einer Pathogen-Pflege-Kanülensammelbehältervorrichtung (120 1-n), die kommunikativ mit der elektronischen Administratorvorrichtung gekoppelt ist, einer Ablagerung (602) eines Pathogen-Pflege-Artikels, der mit dem Pathogen-Pflege-Impfstoff-Verabreichungsereignis assoziiert ist, die in einem Fach der Pathogen-Pflege-Kanülensammelbehältervorrichtung platziert ist;
Detektieren, an der Pathogen-Pflege-Kanülensammelbehältervorrichtung (120 1-n), von Näherungsinformationen, die die Nähe der elektronischen Patientenvorrichtung dazu während des Impfstoff-Verabreichungsereignisses bestätigen;
Erzeugen (603), an der Pathogen-Pflege-Vorrichtung (120 1-n), eines Pathogen-Pflege-Datenverzeichnisses, das mit dem identifizierten Benutzer assoziiert ist, umfassend Daten, die (i) die Ablagerung des Pathogen-Pflege-Impfstoff-Verabreichungsartikels, der mit dem Pathogen-Pflege-Ereignis assoziiert ist, die in einem Fach der Pathogen-Pflege-Kanülensammelbehältervorrichtung platziert ist, und (ii) die Nähe der elektronischen Patientenvorrichtung bei dem Pathogen-Pflege-Ereignis verifizieren; Empfangen, an einer Pathogen-Pflege-Verwaltungs-Plattform, PCM-Plattform, (110), des Pathogen-Pflege-Datenverzeichnisses und des Benutzeridentifikationsdatenverzeichnisses;
Verifizieren (604), durch eine Verarbeitungseinheit (114) der PCM-Plattform (110), der Gültigkeit der empfangenen Informationen, indem Informationen, die in dem Pathogen-Pflege-Datenverzeichnis und/oder dem Benutzeridentifikationsdatenverzeichnis enthalten sind, mit Referenzinformationen verglichen werden, wobei das Verifizieren der Gültigkeit des Pathogen-Pflege-Ereignisses ferner das Prüfen (6044) der Näherungsinformationen und das Prüfen (6045) der Impfbestätigung beinhaltet; und Aktualisieren (607), durch die Verarbeitungseinheit (114), basierend auf der Gültigkeit der empfangenen Informationen, eines digitalen Gesundheitsverzeichnisses, das mit dem Benutzer assoziiert ist.

14. Pathogen-Pflege-Verwaltungssystem (100) gemäß Anspruch 1, das ein Impfstoff-Verabreichungs-Verifikations-System, VAV-System, beinhaltet, wobei der Pathogen-Pflege-Artikel einen Impfstoff-Verabreichungsartikel beinhaltet und das Pathogen-Pflege-Ereignis ein Impfstoff-Verabreichungsereignis beinhaltet.

15. Pathogen-Pflege-Verwaltungssystem (100) gemäß Anspruch 1, wobei die Pathogen-Pflege-Softwareanwendung konfiguriert ist, um die Nähe der elektronischen Vorrichtung des Patienten zu bestätigen, umfassend zum Beispiel durch Scannen eines Identifikationscodes, der auf dem Bildschirm der elektronischen Vorrichtung dargestellt wird, die mit einem Benutzer assoziiert ist, oder Paaren durch eine drahtlose Kurzstreckenverbindung.

## Revendications

1. Un système de gestion de soin aux agents pathogènes (100) pour la gestion et le suivi d'événements d'administration de vaccin de soin aux agents pathogènes associés à un utilisateur patient, le système (100) comprenant :
une application logicielle de soin aux agents pathogènes s'exécutant sur au moins un dispositif électronique d'administrateur (150 1-n) associé à un administrateur de l'événement de soin aux agents pathogènes configurée pour identifier et enregistrer un utilisateur avant un événement de soin aux agents pathogènes, l'application logicielle de soin aux agents pathogènes étant configurée pour générer, durant l'opération, un dossier de données d'identification d'utilisateur comprenant des informations d'identification associées à l'utilisateur et une confirmation de proximité d'un dispositif électronique de patient associé à l'utilisateur patient durant l'événement de soin aux agents pathogènes ;
au moins un dispositif de bac à objets pointus ou tranchants de soin aux agents pathogènes (120 1-n) couplé en communication à l'au moins un dispositif électronique d'administrateur (150 1-n), le dispositif de bac à objets pointus ou tranchants (120 1-n) étant configuré pour détecter :
(i) des dépôts d'au moins un article de soin aux agents pathogènes associé à l'événement d'administration de vaccin de soin aux agents pathogènes ;
(ii) la proximité du dispositif électronique de patient par rapport à celui-ci durant l'événement d'administration de vaccin ;
où en réponse à la détection d'un article de soin aux agents pathogènes qui est placé dans un compartiment de stockage du dispositif de bac à objets pointus ou tranchants (120 1-n), l'au moins un dispositif de bac à objets pointus ou tranchants (120 1-n) est configuré pour générer un dossier de données de soin aux agents pathogènes associé à l'utilisateur patient identifié par l'application logicielle de soin aux agents pathogènes incluant des données vérifiant (i) le dépôt de l'article d'administration de vaccin et (ii) la proximité du dispositif électronique de patient ;
une plate-forme de gestion de soin aux agents pathogènes, PCM, (112) couplée en communication à l'au moins un dispositif de bac à objets pointus ou tranchants (120 1-n) et l'au moins un dispositif électronique d'administrateur (150 1-n) pour recevoir des dossiers de données de soin aux agents pathogènes et des dossiers de données d'identification d'utilisateur correspondants, la plate-forme PCM (112) comprenant :
un module de stockage de données (140) stockant une pluralité de dossiers de santé numériques associés à des utilisateurs, chaque dossier de santé numérique comprenant des données de soin aux agents pathogènes incluant : les données de dépôt d'article de soin aux agents pathogènes, les données de proximité de dispositif électronique de patient, et des données d'identification de patient associées à un utilisateur patient unique, et
une unité de traitement (114) configurée pour mettre à jour le dossier de santé numérique associé à l'utilisateur patient identifié avec des informations reçues en provenance du dispositif de bac à objets pointus ou tranchants (120 1-n) et/ou d'au moins un dispositif électronique (150 1-n).

2. Le système de gestion de soin aux agents pathogènes (100) selon la revendication 1, où l'unité de traitement (114) de la plate-forme PCM (110) comprend un module de validation (1142) configuré, avant la mise à jour d'un dossier de santé numérique, pour valider l'événement de soin aux agents pathogènes correspondant en comparant des informations contenues dans les données de dossier de données de soin aux agents pathogènes et/ou le dossier de données d'identification d'utilisateur patient avec des informations de référence récupérées d'une base de données du module de stockage de données (112) et/ou stockées dans le dossier de santé numérique de l'utilisateur patient correspondant, où la vérification de la validité de l'événement de soin aux agents pathogènes comprend en outre le contrôle (6044) des informations de proximité et le contrôle (6045) de la confirmation de vaccination.

3. Le système de gestion de soin aux agents pathogènes (100) selon la revendication 2, où en réponse à un événement de soin aux agents pathogènes valide, le module de traitement (114) est configuré pour mettre à jour un état de santé du dossier de santé numérique d'utilisateur patient et émettre un certificat correspondant, qui est stocké dans le dossier de données de santé numérique de l'utilisateur patient et transmis en outre à une application logicielle d'utilisateur patient s'exécutant sur un dispositif électronique d'utilisateur patient (150 1-n) associé à l'utilisateur patient.

4. Le système de gestion de soin aux agents pathogènes (100) selon la revendication 2 ou 3, où en réponse à un événement de soin aux agents pathogènes invalide, le module de traitement (114) est configuré pour mettre à jour l'état de santé du dossier de santé numérique correspondant avec la notification de rejet.

5. Le système de gestion de soin aux agents pathogènes (100) selon l'une quelconque des revendications précédentes, où l'unité de traitement (114) comprend un module de rappel (1146) configuré pour émettre un ou plusieurs rappels associés à un événement de soin aux agents pathogènes, sur la base d'informations contenues dans les dossiers de santé numériques d'utilisateur correspondants et/ou d'informations reçues en provenance d'un système externe, les rappels étant transmis à un ou plusieurs dispositifs électroniques (150 1-n) associés à un utilisateur patient à une fréquence sélectionnée.

6. Le système de gestion de soin aux agents pathogènes (100) selon la revendication 5, où le module de rappel (1146) est configuré pour ajuster la fréquence de transmission de rappel sur la base d'une classification des rappels en types normal, urgent, et urgence absolue.

7. Le système de gestion de soin aux agents pathogènes (100) de l'une quelconque des revendications précédentes, où le dossier de données de soin aux agents pathogènes comprend des informations obtenues à partir d'une image de l'article de soin aux agents pathogènes et/ou du balayage d'un code d'identification associé à l'article de soin aux agents pathogènes.

8. Le système de gestion de soin aux agents pathogènes (100) de l'une quelconque des revendications précédentes, où l'application logicielle de soin aux agents pathogènes est configurée pour identifier un utilisateur patient sur la base d'un ID biométrique de l'utilisateur patient comprenant au moins une caractéristique biométrique associée à l'utilisateur patient.

9. Le système de gestion de soin aux agents pathogènes (100) de l'une quelconque des revendications précédentes, où l'application logicielle de soin aux agents pathogènes est configurée pour identifier un utilisateur sur la base du balayage d'un code d'identification présenté sur un écran d'un dispositif électronique associé à l'utilisateur patient.

10. Le système de gestion de soin aux agents pathogènes (100) de l'une quelconque des revendications précédentes, où l'unité de traitement (114) comprend un module de gestion de distribution (1141) configuré pour activer la distribution d'un article de soin aux agents pathogènes à partir d'un réceptacle de stockage (170), sur la base d'informations contenues dans au moins le dossier de données d'identification d'utilisateur patient.

11. Le système de gestion de soin aux agents pathogènes (100) de la revendication 10, où le module de gestion de distribution (1141) est configuré pour surveiller un ensemble de paramètres du réceptacle de stockage (170).

12. Le système de gestion de soin aux agents pathogènes (100) de l'une quelconque des revendications précédentes, où le bac à objets pointus ou tranchants comprend :
un compartiment amovible configuré pour recevoir des dépôts d'article de soin aux agents pathogènes ;
au moins un module de capteur (123) configuré pour détecter le dépôt d'article de soin aux agents pathogènes ;
un module d'horodatage configuré pour horodater le dépôt d'article de soin aux agents pathogènes ;
un module de traitement pour générer le dossier de données de soin aux agents pathogènes ; et
un module de communication (125) configuré pour transmettre le dossier de données de soin aux agents pathogènes à la plate-forme PCM (110).

13. Un procédé pour l'inscription au dossier et la validation d'événements d'administration de vaccin de soin aux agents pathogènes associés à des utilisateurs patients, le procédé comprenant :
l'enregistrement (601), au niveau d'un dispositif électronique d'administrateur (150 1-n) exécutant une application logicielle de soin aux agents pathogènes, d'informations d'identification associées à un utilisateur patient avant un événement de soin aux agents pathogènes ;
la génération, par le dispositif électronique (150 1-n), d'un dossier de données d'identification d'utilisateur comprenant des informations d'identification associées à l'utilisateur identifié et une confirmation de proximité d'un dispositif électronique de patient associé à l'utilisateur patient durant l'événement de soin aux agents pathogènes ;
la détection, au niveau d'un dispositif de bac à objets pointus ou tranchants de soin aux agents pathogènes (120 1-n) couplé en communication au dispositif électronique d'administrateur, d'un dépôt (602) d'un article de soin aux agents pathogènes associé à l'événement d'administration de vaccin de soin aux agents pathogènes qui est placé au niveau d'un compartiment du dispositif de bac à objets pointus ou tranchants de soin aux agents pathogènes ;
la détection au niveau du dispositif de bac à objets pointus ou tranchants de soin aux agents pathogènes (120 1-n) d'informations de proximité confirmant la proximité du dispositif électronique de patient par rapport à celui-ci durant l'événement d'administration de vaccin ;
la génération (603), au niveau du dispositif de soin aux agents pathogènes (120 1-n), d'un dossier de données de soin aux agents pathogènes associé à l'utilisateur identifié incluant des données vérifiant (i) le dépôt de l'article d'administration de vaccin de soin aux agents pathogènes associé à l'événement de soin aux agents pathogènes qui est placé au niveau d'un compartiment du dispositif de bac à objets pointus ou tranchants de soin aux agents pathogènes et (ii) la proximité du dispositif électronique de patient à l'événement de soin aux agents pathogènes ;
la réception, au niveau d'une plate-forme de gestion de soin aux agents pathogènes, PCM, (110), du dossier de données de soin aux agents pathogènes et du dossier de données d'identification d'utilisateur ;
la vérification (604), par une unité de traitement (114) de la plate-forme PCM (110), de la validité des informations reçues en comparant des informations contenues dans le dossier de données de soin aux agents pathogènes et/ou le dossier de données d'identification d'utilisateur avec des informations de référence, où la vérification de la validité de l'événement de soin aux agents pathogènes comprend en outre le contrôle (6044) des informations de proximité et le contrôle (6045) de la confirmation de vaccination ; et
la mise à jour (607), par l'unité de traitement (114) sur la base de la validité des informations, reçues, d'un dossier de santé numérique associé à l'utilisateur.

14. Le système de gestion de soin aux agents pathogènes (100) selon la revendication 1, comprenant un système de vérification d'administration de vaccin, VAV, où l'article de soin aux agents pathogènes comprend un article d'administration de vaccin et l'événement de soin aux agents pathogènes comprend un événement d'administration de vaccin.

15. Le système de gestion de soin aux agents pathogènes (100) selon la revendication 1, où l'application logicielle de soin aux agents pathogènes est configurée pour confirmer la proximité du dispositif électronique du patient y compris par exemple par balayage d'un code d'identification présenté au niveau de l'écran du dispositif électronique associé à un utilisateur ou appariement via une connexion sans fil à courte portée.
